# EUROPEAN PATENT APPLICATION

(11) **EP 2 397 187 A1**
(43) Date of publication of application: **21.12.2011**
(21) Application number: 11004877.4
(22) Date of filing: 15.06.2011
(51) Int. Cl.: A61N 1/40

(54) **Electromagnetic stimulator device and method thereof**

(30) Priority: 21.06.2010 TW 99120125
(71) Applicant: LIN, Yen-Lung, TAIPEI CITY (CN); LO, Wei-Yen, Taiwan 60644 (CN); YEN, Shih-Tu, Taipei County (CN)
(72) Inventor: LIN, Yen-Lung, TAIPEI CITY (CN); LO, Wei-Yen, Taiwan 60644 (CN); YEN, Shih-Tu, Taipei County (CN)
(74) Representative: González Ballesteros, Pedro

(57) **Abstract**

The present provides an electromagnetic stimulation device and a method thereof. Energy stored in a magnetic field can be released in a short period of time when an input current is stopped so as to output an electromagnetic pulse with high voltage and high current by self-inductance of an inductor in order to stimulate organisms. The directions of the current and magnetic field in the major stimulating stage are opposite to that of the input current and magnetic field. The electromotive force in the major stimulating stage is higher than the electromotive force produced by the input source.

## Description

The present invention relates to an electromagnetic stimulation device and method thereof, and more particularly to utilizing the effect of self-inductance to provide an electromagnetic stimulation device and method thereof for organisms.

### BACKGROUND OF THE INVENTION

The stimulative signal generated by nerve can be regarded as an electrical signal, which is used for stimulate various parts of the human body (e.g. organs, tissues, muscles, etc.) to induce corresponding actions or stop action.

Nowadays a machine to stimulate various parts of an organism by electric current or magnetism is usually called an electrotherapy installment or electromagnetic stimulation device, and the method for stimulating an animal body by the electrotherapy installment or electromagnetic stimulation device is generally called electromagnetic stimulation method.

In the present market there are two kinds of non-invasive electromagnetic stimulation device and method thereof:
(1) An electrotherapy installment by utilizing electrode to contact the target portion and then input electric current to the target portion, e.g. Electronic Acupuncture (EA), Functional Electrical Stimulation (FES) and Transcutaneous Electrical Nerve Stimulation (TENS). This kind of device is mainly used for therapy and rehabilitation.
(2) A non-contact electromagnetic stimulation device by controlling an electric current through a coil and then generate magnetic field to stimulate the target portion, e.g. Transcranial Magnetic Stimulation (TMS). This kind of device is mainly used for generating nerve potential for action and for researching the use of nerve.

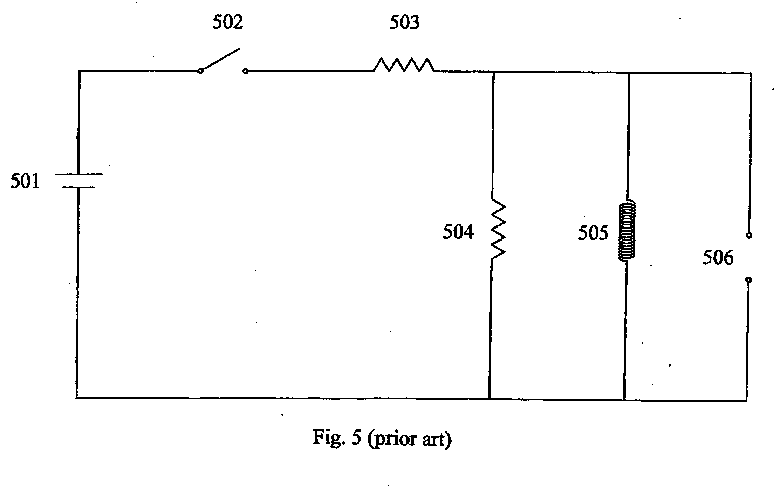

The above-mentioned electromagnetic stimulation devices and methods do not utilize self-inductance for generating instantaneous high voltage and high current to stimulate organism. In the prior art, the best example of utilizing self-inductance is the Spark Ignition (SI) system of automobile or motorcycle. Referring to Fig. 5, a circuit diagram for Spark Ignition (SI) system, during compression stroke of gasoline engine an electric arc is forced to across the gap between plugs for generating sparks to ignite fuel. It must cooperate with the rotation speed and load of the engine to ignite fuel at the right time.

As shown in Fig. 5, the circuit diagram for Spark Ignition (SI) system comprises power supply 501; switch 502 connected to power supply 501; resistor 503 connected serially with switch 502; boosting resistor 504, inductor 505 and gap 506 connected parallelly with power supply 501. Gap 506 is a general term which means that no any component is connected between the two terminals, e.g. gap 506 can be air.

Firstly let switch 502 close, then power supply 501 inputs current through resistor' 503. Since the resistances of boosting resistor 504 and gap 506 are much larger than the resistance of inductor 505, the input current will flow through inductor 505 only, and then return to power supply 501 to form a loop. Then let switch 502 open, the current flow through inductor 505 will flow through the boosting resistor 504 to form a loop, so the voltage across the boosting resistor 504 will go up abruptly, and let the gap 506 discharge for generating sparks.

### SUMMARY OF THE INVENTION

Conventional electrotherapy installment needs an electrode paster for attaching, on the skin of a human body. The stimulating is only effective on the surface layer of the skin and outer layer of the muscle. An electromagnetic stimulation device with higher frequency can stimulate into deeper portion, but due to the electrode paster is attached on the skin surface of the human body, if the current is excessively high, it is dangerous to the human body; if the current is too low, then no therapy effect is achieved. The stimulating area of the electrode paster is limited to the attached portion, so electrotherapy installment for home use only cures the ache of muscles and so on. Even if an implanted electrode can therapy deeper or into some special portions, a surgery is needed for implanting electrode and may cause infecting, failure or damage.

Conventional electromagnetic stimulation device needs a very high power voltage to produce a high current in order to generate effective stimulation to the brain or nerve. But if the voltage and current are too high, the power consumption is high to cause a coil overheated, therefore it is not suitable for home use, and is dangerous for non-expert operation. In addition, this kind of electromagnetic stimulation device is very expensive, overweighted and too big, so it can only be used in hospitals.

The present invention utilizes the effect of self-inductance to provide an electromagnetic stimulation device and method for organisms. The electromagnetic stimulation device according to the present invention has small volume and weight, so it is very useful in daily life. In the present invention, an open of a switch makes related current decreasing rapidly to cause the collapse of the magnetic field and produce a very high induced electromotive force to influence organisms, for example, the very high induced electromotive force can simulate the stimulating electric signal of nerve to stimulate the molecules, organs and tissues in an organism.

The electromagnetic stimulation device according to one embodiment of the present invention comprises: a power supply unit for rectifying and filtering an outside power source to provide a DC current; a voltage converting unit for connecting with the power supply unit to convert the DC current into a rated voltage for the electromagnetic stimulation device; a control unit connected with the voltage converting unit; an energy storage element connected with the voltage converting unit; a switch for controlling the direction of current, with one end to be connected with the energy storage element and the other end to be controlled by the control unit; and an output coil for connecting with the energy storage element by the switch for controlling the direction of current.

By means of the control unit to control the switch for controlling the direction of current to open and close rapidly, the varied current between the output coil and the energy storage element will generate a very high induced electromotive force in the vicinity of the output coil.

The high induced electromotive force will produce a very high stimulating current in a target portion, but the input power voltage is not necessary to be high.

Therefore, the present invention decreases operating danger, electricity consumption, volume and weight, cost of materials of an electromagnetic stimulation device, and is very useful for home use.

The electromagnetic stimulation method according to one embodiment of the present invention comprises steps as below:
(a) a power supply unit is connected with an outside power source for rectifying and filtering AC current of the outside power source into a DC current;
(b)a voltage converting unit converts the DC current of the power supply unit into a rated voltage for the electromagnetic stimulation device;
(c)a switch for controlling the direction of current is connected between a storage element and an output coil, a control unit keeps the switch for controlling the direction of current to open, so the DC current with rated voltage flows into the energy storage element for storaging therein;
(d)the control unit controls the switch for controlling the direction of current to close, so that the DC current flows to the output coil for grounding, and a first high induced magnetic field will be generated in the vicinity of the output coil to produce a first high induced current in a target portion in the vicinity of the output coil for stimulating; and
(e) the control unit controls the switch for controlling the direction of current to open, so as to cause the collapse of the magnetic field in the output coil and produce an induced current opposite to the current of the power supply, thus a second high induced magnetic field is generated to produce a second high induced current in the target portion for stimulating the target portion in the vicinity of the output coil again.

The first induced magnetic field and the second induced magnetic field are opposite in direction. The stimulating time of the second induced magnetic field is shorter than the stimulating time of the first induced magnetic field, while the variation of the second induced voltage and current is higher than the variation of the first induced voltage and current.

### BRIEF DESCRIPTION OF THE DRAWINGS

Fig. 1 shows the block diagram of an electromagnetic stimulation device according to the present invention.
Fig. 2A and Fig. 2B shows the schematic diagrams of a switch for controlling the direction of current according to the present invention.
Fig. 3 shows the flow chart of the electromagnetic stimulation device according to the present invention.
Fig. 4A is a diagram of curve showing the input voltage and output voltage of the output coil of electromagnetic stimulation device according to the present invention varies with time.
Fig. 4B is a diagram of curve showing the input current and output current of the output coil of electromagnetic stimulation device according to the present invention varies with time.
Fig. 5 shows a circuit diagram for Spark Ignition (SI) system,

### DETAILED DESCRIPTIONS OF THE PREFERRED EMBODIMENTS

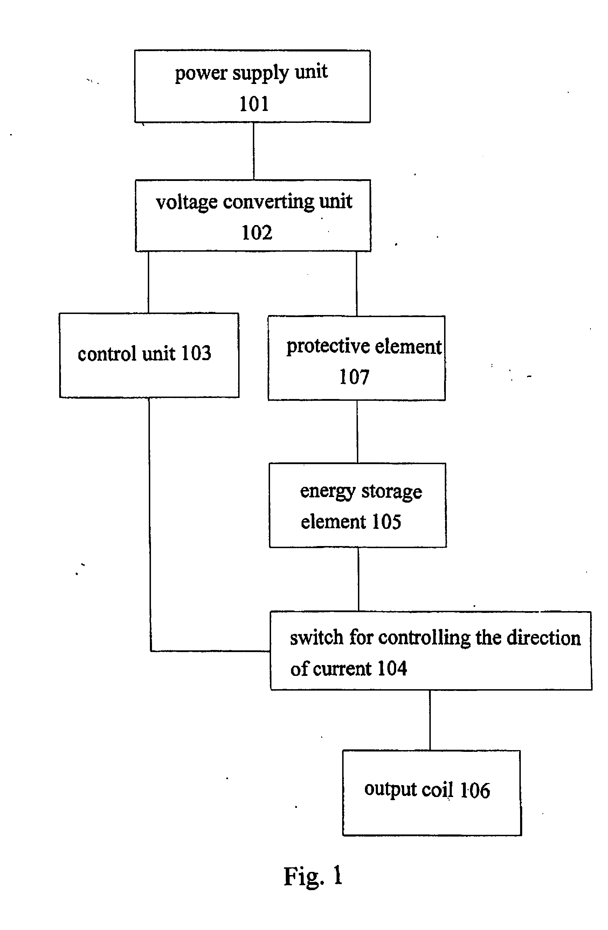

Referring to Fig. 1, which is the block diagram of an electromagnetic stimulation device according to the present invention.

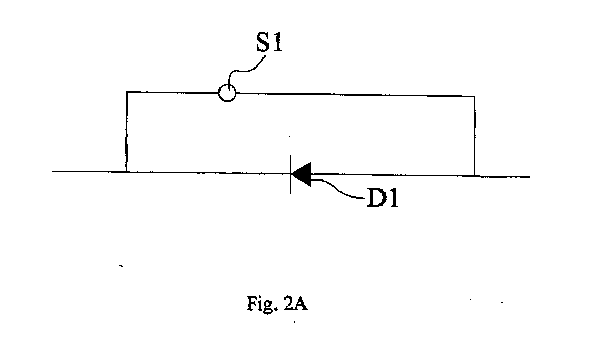

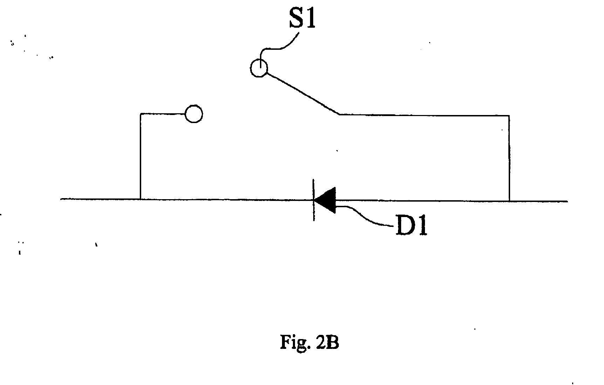

The electromagnetic stimulation device comprises a power supply unit 101 for rectifying and filtering an outside power source (not shown) to provide a useful DC current; a voltage converting unit 102 for connecting with the power supply unit 101 to convert the DC current into a rated voltage of the electromagnetic stimulation device; a control unit 103 connected with the voltage converting unit 102; an energy storage element 105 connected with the voltage converting unit 102; a switch for controlling the direction of current 104, with one end to be connected with the energy storage element 105 and the other end to be controlled by the control unit 103; and an output coil 106 for connecting with the energy storage element 105 by the switch for controlling the direction of current 104. The switch for controlling the direction of current 104 is used to maintain the current flowing in only one direction. In the present embodiment, as shown in Fig. 2A and Fig. 2B, the switch for controlling the direction of current 104 comprises a diode D1 paralleled connected with a switch S1. The control unit 103 controls the switch S1 to open or close. At the beginning the control unit 103 controls the switch S1 to open (as shown in Fig. 2B), i.e. at the beginning current will flow into the energy storage element 105 for storaging therein. In the present embodiment, the energy storage element 105 can be a capacitor, an inductor or a battery. The output coil 106 is an inductor.

After the electromagnetic stimulation device is connected with the outside power source, the power supply unit 101 rectifies and filters the AC current of the outside power source, and then provides a DC current to the voltage converting unit 102. The voltage converting unit 102 converts the DC current into a rated voltage for the electromagnetic stimulation device.

At first, the switch for controlling the direction of current 104 is open, i.e. let the current flow into the energy storage element 105 for storaging therein, and then the control unit 103 controls the switch for controlling the direction of current 104 to close, so that the output coil 106 and the energy storage element 105 are in conducting status, and the current flows from the energy storage element 105 to the output coil 106 for grounding. Since the current in the output coil 106 is zero at the beginning, after the output coil 106 is connected with the energy storage element 105, the current varies with time, so the magnetic field in the vicinity of the output coil 106 also varies. Let the output coil 106 approach a target portion, the varing magnetic field will stimulate the target portion in the vicinity of the output coil 106.

Thereafter, the control unit 103 controls the switch for controlling the direction of current 104 to open, so that the current in the output coil 106 decreases abruptly, and the magnetic field in the vicinity of the output coil 106 also shrinks rapidly to collapse, causing a high induced electromotive force to make the diode D1 of the switch for controlling the direction of current 104 to conduct, therefore the high voltage current returns to the energy storage element 105; and because the resistance of the output coil 106 is very low, the high induced electromotive force will generate high induced current in the vicinity of the output coil 106 to stimulate again the target portion in the vicinity of the output coil 106. The present invention utilizes the mechanism of collapse of the magnetic field to generate high voltage and current, it saves electricity as compared with the prior art. A protective element 107 can be placed between the energy storage element 105 and the voltage converting unit 102 for preventing the induced current from flowing back to the voltage converting unit 102. The protective element 107 can be a diode or a switch.

Repeating the step of the control unit 103 controls the switch for controlling the direction of current 104, high induced current will be intermittently generated in the output coil 106, therefore the target portion in the vincinity of the output coil 106 will be stimulated intermittently. The output coil 106 can be wound into special shape to increase focusing, such as 0 type, 8 types, J type, H type, butterfly type, spring type or four-leaved clover type.

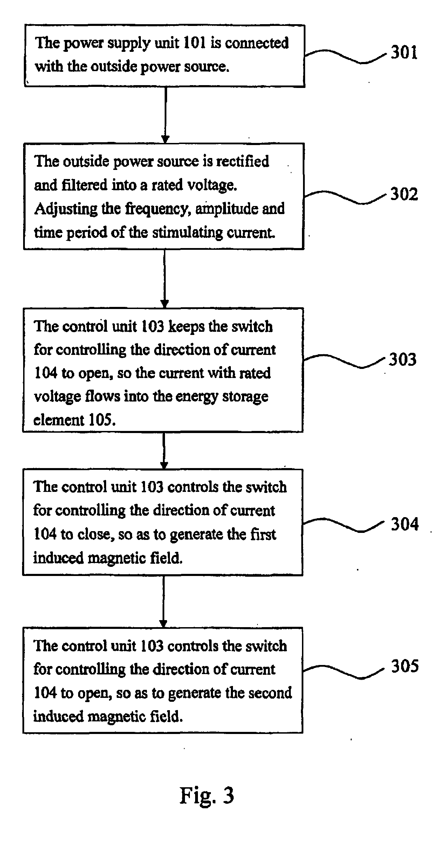

The flow chart of the embodiment of the electromagnetic stimulation method according to the present invention is shown in Fig. 3. As shown in Fig. 3, in step 301, the power supply unit 101 is connected with the outside power source for rectifying and filtering the AC current of the outside power source; in step 302, the voltage converting unit 102 converts the DC current into a rated voltage for the electromagnetic stimulation device, in addition, the electromagnetic stimulation device has an operating interface for adjusting the frequency, amplitude and time period of the stimulating current; in step 303, the control unit 103 keeps the switch for controlling the direction of current 104 to open, so the current flows into the energy storage element 105; in step 304, the control unit 103 controls the switch for controlling the direction of current 104 to close, so the current flows to the output coil 106 for grounding. The first induced magnetic field will be generated in the vicinity of the output coil 106 to produce an induced current in the target portion for stimulating the target portion; in step 305, the control unit 103 controls the switch for controlling the direction of current 104 to open, this will produce a second high induced magnetic field in the output coil 106, and a second induced current will be generated in the target portion in the vicinity of the output coil 106 again for stimulating the target portion. The first induced magnetic field and the second induced magnetic field are opposite in direction. The voltage and the current of the second high induced current are higher than the voltage and current of the outside power source, and the directions of the current and the magnetic field of the second high induced current are opposite to the direction of the input current and magnetic field. But the time period of the input current of the outside power source is longer than the stimulating time of the second high induced current. In a predetermined time period, the steps 304 and 305 are repeated intermittently so that the target portion in the vicinity of the output coil 106 is stimulated intermittently by the high induced current to achieve an expected effect, such as stomach peristalsis, stimulating vasoconstriction, helping cell proliferation, etc.

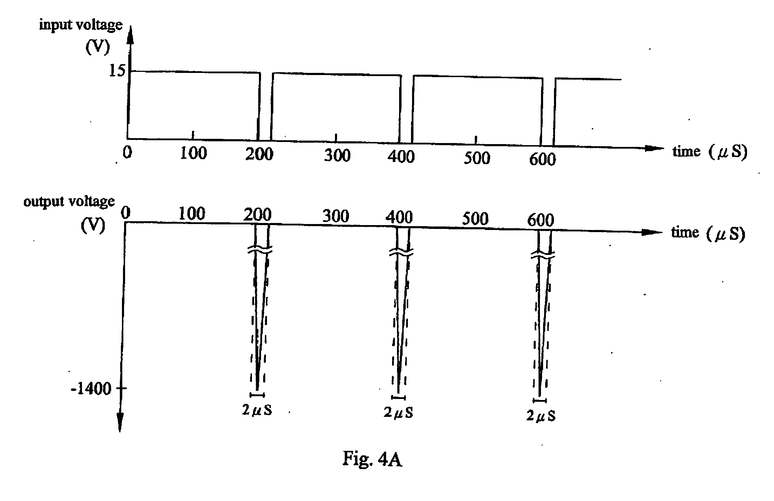

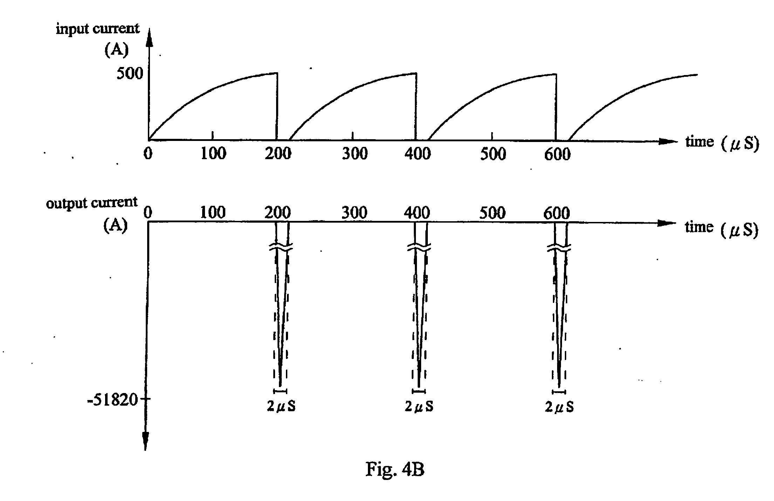

Referring to Fig. 4A and Fig. 4B, experiments of electromagnetic stimulation device according to the present invention are shown. The input voltage and output voltage of the output coil 106 varies with time (Fig. 4A), and the input current and output current of the output coil 106 varies with time (Fig. 4B). The output voltage and output current are 100 times of the input voltage and input current approximately (positive and minus signs mean that the directions of the input and output are opposite). This experiment proves that low input voltage and low input current can provide high output voltage and high output current according to the present invention.

Moreover, for safety the time for the first current stimulation is not limited, but the second current stimulation must not longer than 1ms, the intensity of the electric field is between 1 watt/cm² ∼ 10⁹ watt/cm², the intensity of the magnetic field is between 1 mili-Tesla - 1000 Tesla.

The scope of the present invention depends upon the following claims, and is not limited by the above embodiments.

## Claims

1. An electromagnetic stimulation device, **characterized in that** the electromagnetic stimulation device comprises: a power supply unit for rectifying and filtering of an outside power source to provide a DC current; a voltage converting unit for connecting with the power supply unit to convert the DC current into a rated voltage for the electromagnetic stimulation device ; a control unit connected with the voltage converting unit; an energy storage element connected with the voltage converting unit; a switch for controlling the direction of current, with one end to be connected with the energy storage element and one other end to be controlled by the control unit; and an output coil for connecting with the energy storage element by the switch for controlling the direction of current; at the beginning, the switch for controlling the direction of current is open, and then the control unit controls the switch for controlling the direction of current to close, thereafter the control unit controls the switch for controlling the direction of current to open, so as to generate high induced current in the output coil for stimulating a target portion in the vicinity of the output coil.

2. The electromagnetic stimulation device according to claim 1, **characterized in that** the output coil is an inductor.

3. The electromagnetic stimulation device according to claim 1, **characterized in that** the energy storage element is a capacitor, an inductor or a battery.

4. The electromagnetic stimulation device according to claim 1, **characterized in that** a protective element placed between the energy storage element and the voltage converting unit for preventing the induced current from flowing back to the voltage converting unit.

5. The electromagnetic stimulation device according to claim 4, **characterized in that** the protective element is a diode or a switch.

6. The electromagnetic stimulation device according to claim 1, **characterized in that** the switch for controlling the direction of current comprises a diode paralleled connected with a switch.

7. An electromagnetic stimulation method, **characterized in that** the electromagnetic stimulation method comprises steps as below:(a) a power supply unit is connected with an outside power source for rectifying and filtering AC current of the outside power source into a DC current; (b) a voltage converting unit converts the DC current of the power supply unit into a rated voltage for the electromagnetic stimulation device;(c) a switch for controlling the direction of current is connected between a storage element and an output coil, a control unit keeps the switch for controlling the direction of current to open, so the DC current with rated voltage flows into the energy storage element, (d) the control unit controls the switch for controlling the direction of current to close, so that the DC current flows to the output coil for grounding, and a first high induced magnetic field will be generated in the vicinity of the output coil to produce a first high induced current in a target portion in the vicinity of the output coil for stimulating; and the control unit controls the switch for controlling the direction of current to open, causing a collapse of the magnetic field in the output coil, and produce a second high induced current to stimulate the target portion in the vicinity of the output coil again.

8. The electromagnetic stimulation method according to claim 7, **characterized in that** further comprising a step for adjusting frequency, amplitude and time period of a stimulating current.

9. The electromagnetic stimulation method according to claim 7, **characterized in that** a stimulation time of the second high induced current must not longer than 1ms, an intensity of a electric field is between 1watt/cm² ∼ 10⁹watt/cm², an intensity of a magnetic field is between 1 mili-Tesla - 1000 Tesla.

10. The electromagnetic stimulation method according to claim 7, **characterized in that** a voltage and a current of the second high induced current is higher than a voltage and a current of the outside power source.

11. The electromagnetic stimulation method according to claim 7,' **characterized in that** time period of the DC current flowing into the energy storage element is longer than time period of the second high induced current.
